# EUROPEAN PATENT APPLICATION

(11) **EP 1 880 723 A1**
(43) Date of publication of application: **23.01.2008**
(21) Application number: 06117276.3
(22) Date of filing: 14.07.2006
(51) Int. Cl.: A61K 31/519, A61K 31/436, A61P 35/00, A61P 17/06

(54) **Combination of mTOR inhibitor and antifolate compound**

(71) Applicant: Novartis AG, 4056 Basel (CH)
(72) Inventor: The designation of the inventor has not yet been filed

(57) **Abstract**

Use of a combination of an mTOR inhibitor and an antifolate compound.

## Description

The present invention relates to organic compopunds, such as a combination of pharmaceutically active organic compounds, e.g. a combination of antifolate compounds and mTOR inhibitors.

An mTOR inhibitor is a compound which targets intracellular mTOR ("mammalian Target of rapamycin"). mTOR is a family member of phosphatidylinositol 3-kinase(P13-kinase) related kinase. The compound rapamycin and other mTOR inhibitors inhibit the mTOR pathway via a complex with its intracellular receptor FKBP12 (FK506-binding protein 12).

Rapamycin is a known macrolide antibiotic produced by Streptomyces hygroscopicus. Other mTOR inhibitors include substituted rapamycin, e. g. rapamycin substituted in position 40 and/or 16 and/or 32, for example a compound of formula 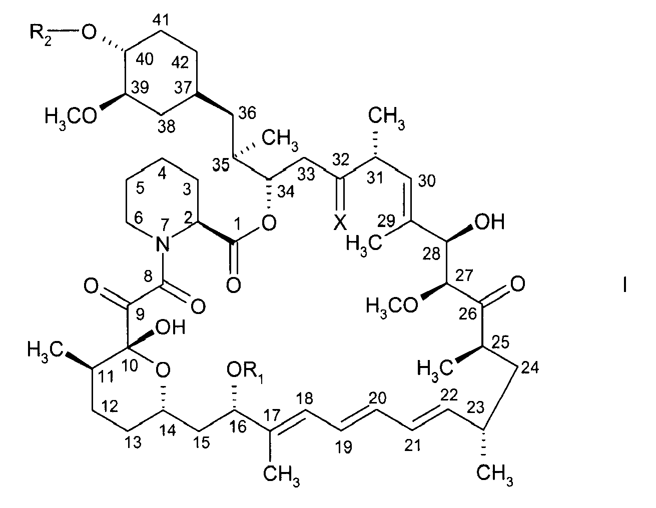 wherein
R₁ is CH₃ or C₃₋₆alkynyl,
R₂ is H, -CH₂-CH₂-OH, 3-hydroxy-2-(hydroxymethyl)-2-methyl-propanoyl or tetrazolyl, and
X is =O, (H, H) or (H, OH), provided that R₂ is other than H when X is =O and R₁ is CH₃, or a prodrug thereof when R₂ is-CH₂-CH₂-OH, e. g. a physiologically hydrolysable ether thereof, for instance -CH₂-CH₂-O-(C₁₋₈)alkyl.
Representative examples of compounds of formula I include e. g. 32-deoxorapamycin, 16-O-substituted rapamycins such as 16-pent-2-ynyloxy-32-deoxorapamycin, 16-pent-2-ynyloxy-32 (S or R) -dihydro-rapamycin, 16-pent-2-ynyloxy-32 (S orR)-dihydro-40-O-(2-hydroxyethyl)-rapamycin, 40-[3-hydroxy-2- (hydroxy- methyl)-2-methylpropanoate]-rapamycin (also known as CCI779), 40-epi-(tetrazolyl)-rapamycin (also known as ABT578), the so-called rapalogs, e. g. as disclosed in W09802441, W00114387 and W00364383, e. g. AP23573, AP23464, AP23675 or AP23841 and compounds disclosed under the name TAFA-93, biolimus-7 or biolimus-9.
A preferred compound is e. g. 40-O-(2-hydroxyethyl)-rapamycin disclosed in Example 8 in WO9409010 (referred hereinafter as Compound A), or 32-deoxorapamycin or 16-pent-2-ynyloxy-32 (S) -dihydro- rapamycin as disclosed in WO9641807, or a compound as disclosed in WO9516691.
Further examples of other mTOR inhibitors are e.g. disclosed in W02004101583, WO9205179, W09402136, W09402385, W09613273.

Preferred mTOR inhibitors include rapamycin, 40-O-(2-hydroxyethyl)-rapamycin, 32-deoxorapamycin, 16-pent-2-ynyloxy-32-deoxorapamycin, 16-pent-2-ynyloxy-32(S or R)-dihydro-rapamycin, 16-pent-2-ynyloxy-32(S orR)-dihydro-40-O-(2-hydroxyethyl)-rapamycin, 40-[3-hydroxy-2- (hydroxy- methyl)-2-methylpropanoate]-rapamycin (also known as CC1779), 40-epi-(tetrazolyl)-rapamycin (also known as ABT578), the so-called rapalogs, e. g. as disclosed in WO9802441, W00114387 and W00364383, e. g. AP23573, AP23464, AP23675 or AP23841 and compounds disclosed under the name TAFA-93, biolimus-7 or biolimus-9, such as
40-O-(2-hydroxyethyl)-rapamycin, and/or
32-deoxorapamycin, and/or
16-pent-2-ynyloxy-32-deoxorapamycin, and/or
16-pent-2-ynyloxy-32-(S or R)-dihydro-rapamycin, and/or
16-pent-2- ynyloxy-32-(S orR)-dihydro-40-0- (2-hydroxyethyl)-rapamycin, and/or
40- [3-hydroxy-2- (hydroxy- methyl)-2-methylpropanoate]-rapamycin (also known as CCI779) and/or
40-epi-(tetrazolyl)- rapamycin (also known as ABT578), and/or
the so-called rapalogs, e. g. AP23573.

mTOR inhibitors, on the basis of observed activity, have been found to be useful as pharmaceuticals, e. g. as immunosuppressants, for example for the treatment of conditions following transplantation; as anti-inflammatory compounds, e.g. for the treatment of IBD, RA; as anti-allergic compounds, e.g. for the treatment of psoriasis, and have additionally potent antiproliferative properties which make them useful for cancer chemotherapy, such as for the treatment of solid tumors, especially of advanced solid tumors.

Antifolate compounds have an inhibitory effect on one or more enzymes which utilize folic acid, and in particular metabolic derivatives of folic acid, as a substrate.
Antifolate compounds are known and e.g. include compounds as described in US5344932, for example including a compound of formula 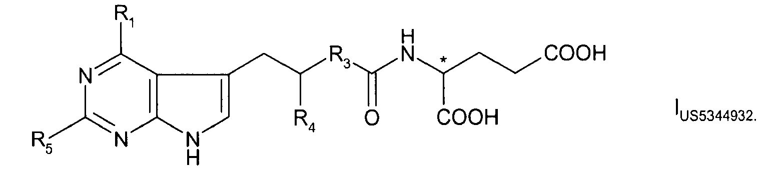 wherein
R₁ is -OH or -NH₂;
R₃ is 1,4-phenylene or 1,3-phenylene unsubstituted or substituted with chloro, fluoro, methyl, methoxy, or trifluoromethyl; thienediyl or furanediyl unsubstituted or substituted with chloro, fluoro, methyl, methoxy, or trifluoromethyl; cyclohexanediyl; or alkanediyl;
R₄ is hydrogen, methyl, or hydroxymethyl; and
R₅ is hydrogen, alkyl of 1 to 6 carbon atoms, or amino;
e.g. the configuration of the substituent at the carbon atom designated * is S;
e.g. in the form of a pharmaceutically acceptable salt;
such as the compound of formula 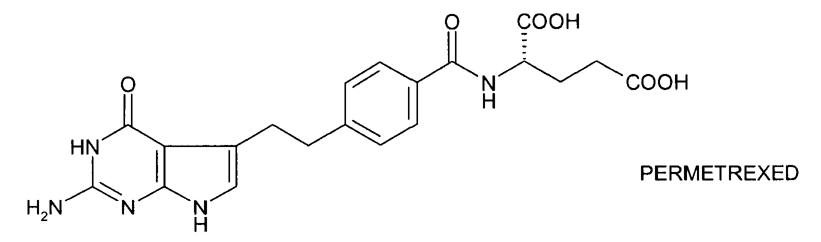 which is known under the name pemetrexed and which is registrated for injection in the form of a disodium salt, heptahydrate under the trade name Alimta®.
Antifolate compounds of formula I appear to be particularly active as inhibitors of thymidylate synthetase, which catalyses the methylation of deoxyuridylic acid to deoxythymidylic acid utilizing N⁵,N¹⁰-methylidenetetrahydrofolate as a coenzyme. The compounds thus can be used to inhibit the growth of those neoplasms which otherwise depend upon the inhibited enzyme.

The compounds of US5344932 are described to inhibit the growth of neoplasms including choriocarcinoma, leukemia, adenocarcinoma of the female breast, epidermid cancers of the head and neck, squamous or small-cell lung cancer, and various lymphosarcomas. The compounds can also be used to treat mycosis fungoides and psoriasis.
Specifically Alimta® is described for the treament of (malignant) mesothelioma, which is a cancer of mesothelial cells, e.g. which cancer can developed in the pleura, (pleural mesothelioma), abdomen, e.g. mesothelioma of the tissues lining the abdominal cavity (peritoneal mesothelioma), and the lining of the lungs, or in the lining of the reproductive organs (begnin mesothelioma), recurrent non-small cell lung cancer (NSCLC), gestational trophoblastic tumor.

A limitation to the development of antifolate compounds is that the cytotoxic activity and subsequent effectiveness of antifolates may be associated with substantial toxicity for some patients. Additionally antifolates as a class are associated with sporadic severe mylosuppression with gastrointestinal toxicity which, though infrequent, carries a high risk of mortality. The inability to control these toxicities led to the abandonment of clinical development of some antifolates and has complicated the clinical development of others.

It was now found that a combination of an mTOR inhibitor and an antifolate, e.g. a compound of formula I_{US5344932}, shows surprising results in the treatment of conditions for which mTOR inhibitors and/or a compound of formula I_{US5344932} are useful, e.g. for the treatment of inflammatory or allergic conditions, such as e.g. psoriasis and for the treatment of cancer, e.g. in the treatment of tumors.

Surprisingly certain toxic effects caused by antifolates of formula I_{US5344932}, may be reduced by the presence of an mTOR-inhibitor, without adversely affecting therapeutic efficacy. Surprisingly a combined treatment with a compound of formula I_{US5344932} and an mTOR inhibitor synergistically may reduce the amount of each single drug, if administered in combination, compared with single treatment for achieving similar effects. Surprisingly the combination of an mTOR inhibitor and a compound of formula I_{US5344932} may reduce the toxic events associated with administration of antifolate drugs of formula I_{US5344932}, e.g. synergistically.

Combinations include fixed combinations, in which two or more pharmaceutically active agents, e.g. an mTOR inhibitor and a compound of formula I_{US5344932}, are in the same formulation; kits, in which two or more pharmaceutically active agents, e.g. an mTOR inhibitor and a compound of formula I_{US5344932}, in separate formulations are sold in the same package, e.g. with instruction for co-administration; and free combinations in which two or more pharmaceutically active agents, e.g. an mTOR inhibitor and a compound of formula I_{US5344932}, are packaged separately, but instruction for simultaneous or sequential administration are given.

In different aspects the present invention provides
- a pharmaceutical composition comprising an mTOR inhibitor and an antifolate compound, e.g. a compound of formula I_{US5344932} in combination beside pharmaceutically acceptable excipient;
- a pharmaceutical package comprising an mTOR inhibitor and and an antifolate compound, e.g. a compound of formula I_{US5344932}, beside instructions for combined administration;
- pharmaceutical package comprising an mTOR inhibitor beside instructions for combined administration with an antifolate compound, e.g.a compound of formula I_{US5344932};
- a pharmaceutical package comprising an antifolate compound, e.g.a compound of formula I_{US5344932} beside instructions for combined administration with an mTOR inhibitor;
- a combination of an mTOR inhibitor and an antifolate compound, e.g.a compound of formula I_{US5344932};
- a method for treatment of conditions for which mTOR inhibitors and/or an antifolate compound, e.g.a compound of formula I_{US5344932} are useful, e.g. for the treatment of inflammatory and allergic conditions, such as e.g. psoriasis and for the treatment of cancer, e.g. for the treatment of cancer, comprising administering to a subject in need thereof an effective amount of an mTOR inhibitor in combination with an antifolate compound, e.g.a compound of formula I_{US5344932}, e.g. either in sequence or simultanously;
- a method for the preparation of a medicament for the treatment of conditions for which mTOR inhibitors and/or an antifolate compound, e.g. a compound of formula I_{US5344932} are useful, e.g. for the treatment of inflammatory and allergic conditions, such as e.g. psoriasis and for the treatment of cancer, e.g. for the treatment of cancer, comprising combining, e.g. mixing, an mTOR inhibitor and an antifolate compound, e.g.a compound of formula I_{US5344932}, either separately, or commonly, with pharmaceutically acceptable excipient;
- a method for the preparation of a medicament for the treatment of conditions for which mTOR inhibitors and/or an antifolate compound, e.g.a compound of formula I_{US5344932} are useful, e.g. for the treatment of inflammatory and allergic conditions, such as e.g. psoriasis and for the treatment of cancer, e.g. for the treatment of cancer, comprising combining, e.g. mixing, an mTOR inhibitor with pharmaceutically acceptable excipient to obtain a pharmaceutical composition 1, and combining, e.g. mixing, an antifolate compound, e.g.a compound of formula I_{US5344932} with pharmaceutically acceptable excipient to obtiain a pharmaceutical composition 2, and either
   (i) combining the pharmaceutical composition 1 and the pharmaceutical composition 2 in one single package, or
   (ii) packing the pharmaceutical composition 1 separately and packing the pharmaceutical composition 2 separately, but adding to each package instructions for combined administriation of pharmaceutical composition 1 and pharmaceutical composition 2.
- a method of reducing the toxicity associated with the administration of an antifolate compound, e.g. a compound of formula I_{US5344932} to a subject, comprising administering to said subject an effective amount of an antifolate compound, e.g. a compound of formula I_{US5344932} in combination with an effective amount of an mTOR inhibitor.
- a method for improving the therapeutic utility of an antifolate compound, e.g. a compound of formula I_{US5344932}, by administrating to a subject in need of cancer treatment an antifolate compound, e.g. a compound of formula I_{US5344932} and an mTOR inhibitor.

"A method for treating cancer" or "the use in cancer treatment" of a combination a compound of formula I_{US5344932} and an mTOR inhibitor. includes in several aspects
- a method for inhibiting growth of tumors, comprising administering to a subject in need thereof a therapeutical effective amount of an mTOR inhibitor in combination with an antifolate compound, e.g. a compound of formula I_{US5344932},
- a method for inducing tumor regression, e. g. tumor mass reduction, comprising administering to a subject in need thereof a therapeutical effective amount of an mTOR inhibitor in combination with an antifolate compound, e.g. a compound of formula I_{US5344932},
- a method for treating tumor invasiveness or symptoms associated with tumor growth, comprising administering to a subject in need thereof a therapeutically effective amount of an mTOR inhibitor in combination with an antifolate compound, e.g. a compound of formula I_{US5344932},
- a method for preventing metastatic spread of tumors or for preventing or inhibiting growth of micrometastasis, comprising administering to a subject in need thereof a therapeutically effective amount of an mTOR inhibitor in combination with an antifolate compound, e.g. a compound of formula I_{US5344932},.

In another aspect the present invention provides the use of an mTOR inhibitor in combination with an antifolate compound, e.g. a compound of formula I_{US5344932}, in any of a method provided by the present invention.
In another aspect the present invention provides the use of an mTOR inhibitor in combination with an antifolate compound, e.g. a compound of formula I_{US5344932} in any of a combination or pharmaceutical composition, or pharmaceutical package provided by the present invention.

Treatment as used herein includes treatment and prevention.

In combination (treatment) according to the present invention both, an mTOR inhibitor and a compound of formula I_{US5344932}, show pharmaceutical activity.

Cancer treatment as used herein is meant to include the treatment of solid tumors. By"solid tumors" as used herein are meant tumors and/or metastasis (wherever located), e. g. brain and other central nervous system tumors (eg. tumors of the meninges, brain, spinal cord, cranial nerves and other parts of central nervous system, e. g.glioblastomas or medulla blastomas) ; head and/or neck cancer; breast tumors; circulatory system tumors (e. g. heart, mediastinum and pleura, and other intrathoracic organs, vascular tumors and tumor-associated vascular tissue); excretory system tumors (e. g. kidney, renal pelvis, ureter, bladder, other and unspecified urinary organs); gastrointestinal tract tumors (e. g. oesophagus, stomach, small intestine, colon, colorectal, rectosigmoid junction, rectum, anus and anal canal), tumors involving the liver and intrahepatic bile ducts, gall bladder, other and unspecified parts of biliary tract, pancreas, other and digestive organs); head and neck; oral cavity(lip, tongue, gum, floor of mouth, palate, and other parts of mouth, parotid gland, and other parts of the salivary glands, tonsil, oropharynx, nasopharynx, pyriform sinus, hypopharynx, and other sites in the lip, oral cavity and pharynx); reproductive system tumors (e.g. vulva, vagina, Cervixuteri, Corpus uteri, uterus, ovary, and other sites associated with female genital organs, placenta, penis, prostate, testis, and other sites associated with male genital organs); respiratory tract tumors (e. g. nasal cavity and middle ear, accessory sinuses, larynx, trachea, bronchus and lung, e. g. small cell lung cancer or non-small cell lung cancer); skeletal system tumors (e. g. bone and articular cartilage of limbs, bone articular cartilage and other sites); skin tumors (e. g. malignantmelanoma of the skin, non-melanoma skin cancer, basal cell carcinoma of skin, squamous cell carcinoma of skin, mesothelioma, Kaposi's sarcoma); and tumors involving other tissues incluing peripheral nerves and autonomic nervous system, connective and soft tissue, retroperitoneum and peritoneum, eye and adnexa, thyroid, adrenal gland and other endocrine glands and related structures, secondary and unspecified malignant neoplasm of lymph nodes, secondary malignant neoplasm of respiratory and digestive systems and secondary malignant neoplasm of other sites. Where hereinbefore and subsequently a tumor, a tumor disease, a carcinoma or a cancer is mentioned, also metastasis in the original organ or tissue and/or in any other location are implied alternatively or in addition, whatever the location of the tumor and/or metastasis is.
Cancer treatment as used herein is meant to include the treatment of endicrine tumors, Endocrine tumors include neuroendocrine tumors, such as pancreatic neuroendocrine tumors. Carcinoid tumors are neuroendocrine tumors and include carcinoid tumors arising from the foregut, e.g., bronchial or gastric carcinoid; midgut, e.g., small intestine or appendiceal carcinoid tumors; or hindgut, e.g. rectal carcinoid tumors; such as carcinoid tumors of the GI tract. Symptoms of carcinoid cancer include e.g. a carcinoid syndrom. Cancer treatment as used herein is meant to include the inhibition of the growth of neoplasms including choriocarcinoma, leukemia, adenocarcinoma of the female breast, epidermid cancers of the head and neck, squamous or small-cell lung cancer, and various lymphosarcomas.
Cancer treatment as used herein is meant to include treatment of (malignant) mesothelioma, which is a cancer of mesothelial cells, e.g. which cancer can developed in the pleura, (pleural mesothelioma), abdomen, e.g. mesothelioma of the tissues lining the abdominal cavity (peritoneal mesothelioma), and the lining of the lungs, or in the lining of the reproductive organs (begnin mesothelioma), recurrent non-small cell lung cancer (NSCLC), gestational trophoblastic tumor.

A pharmaceutical composition according to the present invention may further comprise another drug, preferably another drug which is appropriate for the treatment of cancer.
Such other drugs e.g. include drugs which are disclosed as "chemotherpeutic agents" in WO02066019, e.g. on pages 5 and 6 under i) to x), in more detail on pages 6 to 11, namely agents which are disclosed to be useful in combination treatment of solid tumors.
Another drug as used herein is meant to include any drug other than an mTOR inhibitor or a compound of formula I_{US5344932}, which may have beneficial effects in a use or a method of the present invention, e.g. including anti-inflammatory and/or an immunomodulatory drugs.
For example, an mTOR inhibitor may be used in combination with a calcineurin inhibitor, e.g. cyclosporin A or FK 506; an ascomycin having immuno-suppressive properties, e.g. ABT-281, ASM981; corticosteroids; cyclophosphamide; azathioprene; 6-mercaptopurine, methotrexate; leflunomide; mizoribine; mycophenolic acid or salt; mycophenolate mofetil; 15-deoxyspergualine or an immunosuppressive homologue, analogue or derivative thereof; a PKC inhibitor, e.g. as disclosed in WO 02/38561 or WO 03/82859, e.g. the compound of Example 56 or 70; a JAK3 kinase inhibitor, e.g. N-benzyl-3,4-dihydroxy-benzylidene-cyanoacetamide α-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550), or a compound as disclosed in WO 04/052359 or WO 05/066156; a S1 P receptor agonist or modulator, e.g. FTY720 optionally phosphorylated or an analog thereof, e.g. 2-amino-2-[4-(3-benzyloxyphenylthio)-2-chlorophenyl]ethyl-1,3-propanediol optionally phosphorylated or 1-{4-[1-(4-cyclohexyl-3-trifluoromethyl-benzyloxyimino)-ethyl]-2-ethyl-benzyl)-azetidine-3-carboxylic acid or its pharmaceutically acceptable salts; immunosuppressive monoclonal antibodies, e.g., monoclonal antibodies to leukocyte receptors, e.g., MHC, CD2, CD3, CD4, CD7, CD8, CD25, CD28, CD40, CD45, CD52, CD58, CD80, CD86 or their ligands; other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA41g (for ex. designated ATCC 68629) or a mutant thereof, e.g. LEA29Y; adhesion molecule inhibitors, e.g. LFA-1 antagonists, ICAM-1 or -3 antagonists, VCAM-4 antagonists or VLA-4 antagonists, CCR9 antagonists, MIF inhibitors, 5-aminosalicylate (5-ASA) agents, such as sulfasalazine, Azulfidine®, Asacol®, Dipentum®, Pentasa®, Rowasa®, Canasa®, Colazal®, e.g. drugs containing mesalamine; e.g mesalazine in combination with heparin; antibodies which bind to TNF-alpha, such as infliximab (Remicade®), antibiotics, such as penicillins, cephalosporins, erythromycins, tetracyclines, sulfonamides, pleuromutilins, fluoroquinolones, e.g. metronidazole, ciprofloxacin; probiotics and commensal bacteria e.g. Lactobacillus, Lactobacillus reuteri; 'multi-functional anti-inflammatory' drugs (MFAIDs), such as membrane-anchored phospholipase A2 inhibitors linked to glycosaminoglycans; antidiarrheal agents, e.g. including diphenoxylate, loperamide, codeine.
For example a compound of formula I_{US5344932} may be used in combination with a methylmalonic acid lowering agent (see e.g. W02002002093).

As used herein, the term "effective amount" refers to an amount of a compound or drug, which is capable of performing the intended result. For example, an effective amount of drugs in combination that are administered in an effort to reduce tumor growth is that amount which is required to reduce tumor growth.
An appropriate dosage regarding Compound A in treatment according to the present invention includes a daily dose of from (about)1 mg up to (about) 20 mg, such as 2.5 mg, 5 mg, or10 mg.daily; and a weekly dosage from (about) 10 mg to (about) 75 mg, such as 20 mg, 30 mg or 50 mg weekly. Such dosing may be continous or interrupted.
Commercially available permetrexed may be administered following standard procedures. Permetrexed may be administered alongside the 21.day cycle of permetrexed, applying standard pre-medication, dose interruption and adjustment.

As used herein, the term "toxicity" refers to a toxic event associated with the administration on an antifolate, see e.g. "Antifolate Drugs in Cancer Therapy", Humana Press.

### Methods

To assess the effect of an mTOR inhibitor on the antitumor efficacy of a compound of in a human tumor xenograft model, female nude mice bearing human MX-1 breast carcinoma may be treated with a compound of formula I_{US5344932} alone, or in combination with an mTOR inhibitor.
The animals may be maintained on sterilized standard lab chow ad libitum and sterilized water ad libitum. The human MX-1 tumor cells (5*10<6>) obtained from donor tumors may be implanted subcutaneously in a thigh of female nude mice 8- to 10-weeks old. Beginning on day 7 post tumor cell implantation, the animals may be treated with a compound of formula I_{US5344932} (100 mg/kg or 150 mg/kg) once daily on days 7 through 11 and 14 through 18 by intraperitoneal injection alone or along with an mTOR inhibitor (oral treatmernt, 1 to 30 mg/kg).
Tumor response may be nonitored tumor volume measurements twice weekly over the course of the experiment. Toxicity may be monitored by body weight measurements made at the same time as the tumor volume measurements. Tumor growth delay is meant to be the difference in days for the treated and the controls tumors to reach 1000 mm.
The human MX-1 breast carcinoma xenograft is responsive to treatment with a compound of formula I_{US5344932} with doses of 100 mg/kg and 150 mg/kg producing tumor growth delays of 17 days and 21 days, respectively. An mTOR inhibitor administered to the animals alone at two doses 5 mg/kg and 20 mg/kg on the same schedule as a compound of formula I_{US5344932} may produce tumor growth delays of several days.
Combinations of a compound of formula I_{US5344932} may be administered along with an mTOR inhibitor as simultaneous combination regimens, or an mTOR inhbitor may be administered before administration of a compound of formula I_{US5344932}, or a compound of formula I_{US5344932}, may be administered before administration of an mTOR inhibitor. Administration of a compound of formula I_{US5344932}, in combination with an mTOR inhibitor may reduce the delay of tumor growth.
Body weight may be used as a general measure of toxicity for each of the treatment regimens. The body weight loss pattern may reflect the treatment regimens with weight decrease during the treatment times of days 7 through 11 and 14 through 18 with some weight recovery during the intervening two days. The weight loss due to a compound of formula I_{US5344932}, may be dose dependent but overall minor. The animals treated with a compound of formula I_{US5344932} and and an mTOR inhibitor may gain weight over the course of the experiment.
The timing of administering a compound of formula I_{US5344932} and an mTOR inhibitor may be varied.

### Cliical trials

Investigated is an open label, multicenter Phase I study investigating the combination of Compound A with the standard treatment regimen of pemetrexed in patients with advanced (unresectable or metastatic) NSCLC previously treated with one line of chemotherapy. A maximum of 120 patients are enrolled.

### Study objectives

To confirm the feasible dose levels/regimens of RAD001 combined with the standard pemetrexed regimen in patients with NSCLC previously treated with one regimen of chemotherapy based on the evaluation of safety and ability to deliver the required dose intensity of pemetrexed

To confirm the PK of RAD001 in NSCLC patients treated with the combination RAD001 and pemetrexed and to estimate the PK interaction between RAD001 and pemetrexed.
To confirm the clinical efficacy of combined administration of different RAD001 dose levels / regimens with standard pemetrexed treatment based on the evaluation of overall tumour response according to RECIST.

The study is designed as a Bayesian sequential dose-escalation scheme based on:
• a time-to-event model of the occurrence of DLTs estimating the probability that patients experience a DLT within their first cycle of treatment ("End-of-Cycle-1 DLT rate")

The study is designed as a Bayesian sequential dose-escalation scheme based on
• a time-to-event model of the occurrence of DLTs estimating the probability that patients experience a DLT within their first cycle of treatment ("End-of-Cycle-1 DLT rate")
and
• a model estimating the probability that the Relative Dose Intensity (RDI) of pemetrexed is optimal *versus* sub-optimal.

Successive dose levels are investigated initially in 2 separate treatment arms, each corresponding to a different dosing schedule for Compound A:
• Arm 1: continuous daily dosing of Compound A in combination with the standard 21-day treatment cycle of pemetrexed
• Arm 2: continuous weekly dosing of Compound A (Days 1, 8, 15) in combination with the standard 21-day treatment cycle of pemetrexed
After the completion of the evaluation of continuous daily regimen of Compound A in arm 1 a further Compound A regimen may be initiated as follows:
• Interrupted daily dosing of Compound A with no administration of Compound A between Days 15 and 21 of each cycle of therapy)
Patients are randomised and centrally allocated to either arm and are enrolled at the current dose level/regimen. Dose escalation can occur in parallel, although independently, in each arm.
RDI is evaluated when the first 15 patients in each arm have been followed for at least 2 cycles and this time point will be considered an interim analysis look. If the RDI evaluation contradicts the dose-escalation decision based on the safety signals, then a second interim look during the study is to be planned. The RDI is evaluated as well at the end of the trial.

The study investigates two different dosing schedules of COMPOUND A (daily and weekly) in combination with the standard 21-day cyclic administration of pemetrexed. Within each COMPOUND A dosing schedule, two different regimens, continuous and interrupted dosing, are investigated and up to three individual dose levels of RAD001 per regimen are explored The Compound A (C-A) doses within the two arms are as follows:

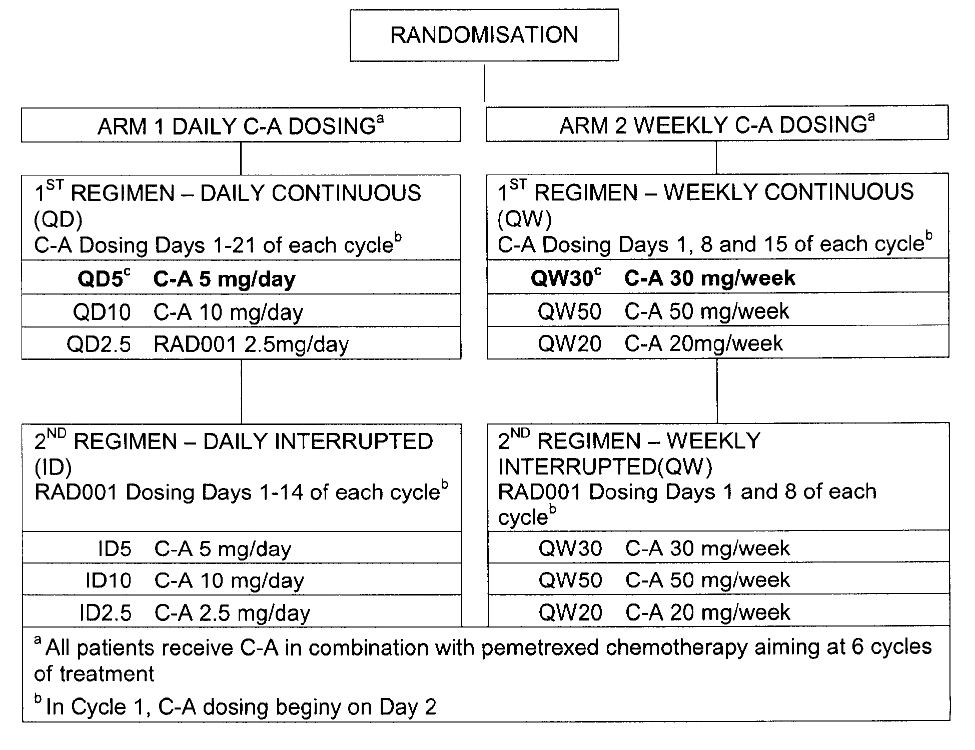

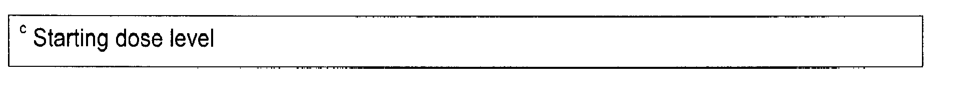

Compound A dosing is commenced on Day 2 of Cycle 1 in both arms, just before pemetrexed infusion. The decision to escalate the dose of Compound A will depend on the estimated end-of-cycle 1 DLT rate and on the RDI of the chemotherapy partner of Compound A. Patients will continue to receive Compound A until progression of disease or unacceptable toxicity.

### Pemetrexed doses

All patients receive their respective Compound A doses alongside the standard 21-day cycle of pemetrexed, applying standard pre-medication, dose interruption and adjustment (see appendix).
• Pemetrexed is administered on Day 1 at a dose of 500 mg/m² as a 10 min. intravenous infusion.
Patients may receive up to a maximum of 6 cycles of pemetrexed.
Endpoints: Dose limiting toxicities occurring at any time, relative dose intentsity of chemotherapy.

The study is aimed at providing the patients six cycles of pemetrexed chemotherapy. One cycle is defined as an interval between two consecutive administrations of chemotherapy regimens (with a default of 21-days) and Day 1 for each cycle is defined as the day of the initiation of the chemotherapy. During each cycle, patients have regular safety laboratory tests on a weekly basis (Days 8 and 15). On Day 1 they will also undergo a full safety assessment before further chemotherapy is initiated.
Only in Cycle treatment with RAD001 is delayed until Day 2 to allow serial blood sampling for the evaluation of baseline PK profiles of pemetrexed on Day 1. The PK blood sampling is continued on Day 8 of Cycle 1 (baseline PK profile of RAD001), and on Day 1 of Cycle 2 (combination PK profiles for both study drugs are administered together).
Computerised tomography (CT) scans for tumour measurements is performed routinely every six weeks (after every 2 cycles of pemetrexed) while the patient is receiving chemotherapy. Following completion or discontinuation of chemotherapy, all patients may continue to receive RAD001 on a continuous daily or weekly basis (irrespective of the RAD001 regimen while on chemotherapy) until progressive disease or unacceptable toxicity occurs. In such cases, CT scans are carried out every 6 weeks.
Efficacy is evaluated using the following endpoints: Overall response rate (ORR) according to RECIST (optionally amended RESIST), early progression rate (EPR) according to RECIST (optionally amended RESIST). Tumor assessment is performed by a CT scan throughout the study Drug-drug interaction is evaluated from the comparison of study levels when administered alone or in combination.

In the clinical trials COMPOUND A in combination with permetrexed show appropriate efficacy regarding tumor growth. The combination of COMPOUND A with permetrexed shows synergistic effects in several aspects.

## Claims

1. A pharmaceutical composition comprising an mTOR inhibitor and an antifolate compound, e.g. a compound of formula 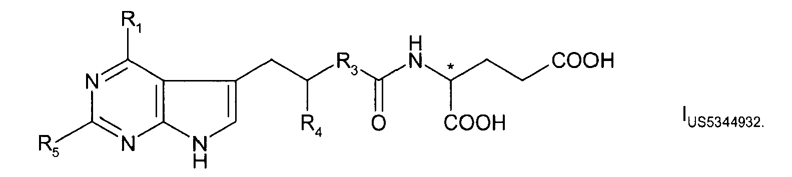 wherein
R₁ is -OH or -NH₂;
R₃ is 1,4-phenylene or 1,3-phenylene unsubstituted or substituted with chloro, fluoro, methyl, methoxy, or trifluoromethyl; thienediyl or furanediyl unsubstituted or substituted with chloro, fluoro, methyl, methoxy, or trifluoromethyl; cyclohexanediyl; or alkanediyl; R₄ is hydrogen, methyl, or hydroxymethyl; and
R₅ is hydrogen, alkyl of 1 to 6 carbon atoms, or amino;
e.g. the configuration of the substituent at the carbon atom designated * is S;
e.g. in the form of a pharmaceutically acceptable salt;
in combination beside pharmaceutically acceptable excipient.

2. A pharmaceutical package comprising an mTOR inhibitor and an antifolate compound, e.g. a compound of formula I_{US5344932} as defined in claim 1, beside instructions for combined administration.

3. - A pharmaceutical package comprising an mTOR inhibitor beside instructions for combined administration with an antifolate compound, e.g. a compound of formula I_{US5344932}; as defined in claim 1.

4. A pharmaceutical package compring an antifolate compound, e.g. a compound of formula I_{US5344932;} as defined in claim 1, beside instructions for combined administration with an mTOR inhibitor.

5. A combination of an mTOR inhibitor and an antifolate compound, e.g. a compound of formula I_{US5344932}; as defined in claim 1.

6. A method for treatment of conditions for which mTOR inhibitors and/or an antifolate compound, e.g. a compound of formula I_{US5344932}; as defined in claim 1are useful, e.g. for the treatment of inflammatory and allergic conditions, such as e.g. psoriasis and for the treatment of cancer, e.g. for the treatment of cancer, comprising administering to a subject in need thereof an effective amount of an mTOR inhibitor in combination with an antifolate compound, e.g. a compound of formula I_{US5344932}; as defined in claim 1, e.g. either in sequence or simultanously;

7. A method for the preparation of a medicament for the treatment of conditions for which mTOR inhibitors and/or an antifolate compound, e.g. a compound of formula I_{US5344932}; as defined in claim 1 are useful, e.g. for the treatment of inflammatory and allergic conditions, such as e.g. psoriasis and for the treatment of cancer, e.g. for the treatment of cancer, comprising combining, e.g. mixing, an mTOR inhibitor and a compound of formula I_{US5344932}; as defined in claim 1, either separately, or commonly, with pharmaceutically acceptable excipient;

8. A method for the preparation of a medicament for the treatment of conditions for which mTOR inhibitors and/or an antifolate compound, e.g. a compound of formula I_{US5344932} are useful, e.g. for the treatment of inflammatory and allergic conditions, such as e.g. psoriasis and for the treatment of cancer, e.g. for the treatment of cancer, comprising combining, e.g. mixing, an mTOR inhibitor with pharmaceutically acceptable excipient to obtain a pharmaceutical composition 1, and combining, e.g. mixing, an antifolate compound, e.g. a compound of formula I_{US5344932} with pharmaceutically acceptable excipient to obtiain a pharmaceutical compoisition 2, and either
(i) combining the pharmaceutical composition 1 and the pharmaceutical composition 2 in one single package, or
(ii) packing the pharmaceutical composition 1 separately and packing the pharmaceutical composition 2 separately, but adding to each package instructions for combined administriation of pharmaceutical composition 1 and pharmaceutical composition 2.

9. A method of reducing the toxicity associated with the administration of an antifolate compound, e.g. a compound of formula I_{US5344932}; as defined in claim 1, to a subject, comprising administering to said subject an effective amount of a compound of formula I_{US5344932}; as defined in claim 1, in combination with an effective amount of an mTOR inhibitor.

10. A method for improving the therapeutic utility of an antifolate compound, e.g. a compound of formula I_{US5344932}; as defined in claim 1, by administrating to a subject in need of cancer treatment a compound of formula I_{US5344932}; as defined in claim 1, and an mTOR inhibitor.

11. A method for inhibiting growth of tumors, comprising administering to a subject in need thereof a therapeutical effective amount of an mTOR inhibitor in combination with an antifolate compound, e.g. a compound of formula I_{US5344932}; as defined in claim 1.

12. A method for inducing tumor regression, e. g. tumor mass reduction, comprising administering to a subject in need thereof a therapeutical effective amount of an mTOR inhibitor in combination with an antifolate compound, e.g. a compound of formula I_{US5344932}; as defined in claim 1.

13. A method for treating tumor invasiveness or symptoms associated with tumor growth, comprising administering to a subject in need thereof a therapeutically effective amount of an mTOR inhibitor in combination with an antifolate compound, e.g. a compound of formula I_{US5344932}; as defined in claim 1.

14. A method for preventing metastatic spread of tumors or for preventing or inhibiting growth of micrometastasis, comprising administering to a subject in need thereof a therapeutically effective amount of an mTOR inhibitor in combination with an antifolate compound, e.g. a compound of formula I_{US5344932}; as defined in claim 1.

15. A pharmaceutical composition, pharmaceutical package, combination or a method according to any preceding claim, wherein an mTOR inhibitor is rapamycin or a compound of formula I 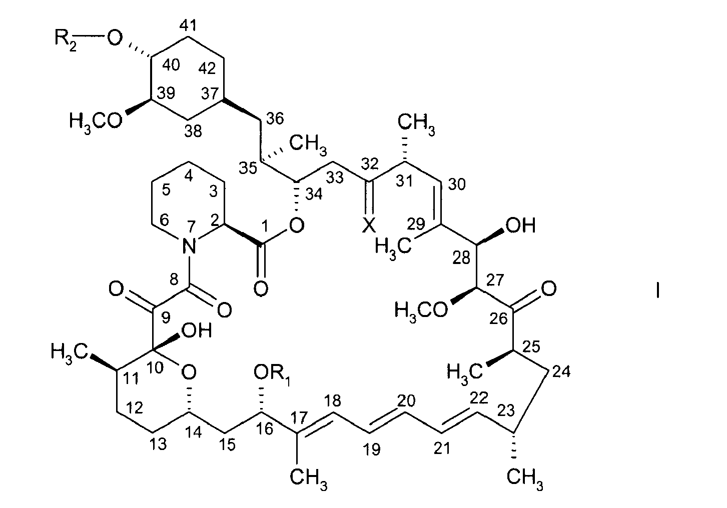 wherein
R₁ is CH₃ or C₃₋₆alkynyl,
R₂ is H,-CH₂-CH₂-OH, 3-hydroxy-2-(hydroxymethyl)-2-methyl-propanoyl or tetrazolyl, and
X is =O, (H, H) or (H, OH), provided that R₂ is other than H when X is =O and R₁ is CH₃, or a prodrug thereof when R₂ is-CH₂-CH₂-OH, e. g. a physiologically hydrolysable ether thereof, for instance -CH₂-CH₂-O-(C₁₋₈)alkyl.

16. A pharmaceutical composition, pharmaceutical package, combination or a method according to claim 15, wherein an mTOR inhibitor is rapamycin, 40-O-(2-hydroxyethyl)-rapamycin, 32-deoxorapamycin, 16-O-substituted rapamycins such as 16-pent-2-ynyloxy-32-deoxorapamycin, 16-pent-2-ynyloxy-32(S or R)-dihydro-rapamycin, 16-pent-2- ynyloxy-32(S or R)-dihydro-40-O-(2-hydroxyethyl)-rapamycin, 40-[3-hydroxy-2-(hydroxy- methyl)-2-methylpropanoate]-rapamycin (also known as CCI779), 40-epi-(tetrazolyl)- rapamycin (also known as ABT578), the so-called rapalogs, e. g. AP23573, AP23464, AP23675 or AP23841, or compounds disclosed under the name TAFA-93, biolimus-7 or biolimus-9.

17. A pharmaceutical composition, pharmaceutical package, combination or a method according to any one of claims 15 or 16, wherein an mTOR inhibitor is rapamycin, 40-O-(2-hydroxyethyl)-rapamycin, 32-deoxorapamycin, 16-pent-2-ynyloxy-32-deoxorapamycin, 16-pent-2-ynyloxy-32(S or R)-dihydro-rapamycin, 16-pent-2-ynyloxy-32(S or R)-dihydro-40-O-(2-hydroxyethyl)-rapamycin, 40-[3-hydroxy-2-(hydroxy-methyl)-2-methylpropanoate]-rapamycin (also known as CCI779), 40-epi-(tetrazolyl)- rapamycin (also known as ABT578), or AP23573.

18. A pharmaceutical composition, pharmaceutical package, combination or a method according to any one of claims 15 or 17, wherein an mTOR inhibitor is 40-0-(2-hydroxyethyl)-rapamycin.

19. A pharmaceutical composition, pharmaceutical package, combination or a method according to any one of claims 15 or 18, wherein an antifolate compound is permetrexed, e.g. of formula
